# EUROPEAN PATENT SPECIFICATION

(11) **EP 3 103 448 B1**
(45) Date of publication and mention of the grant of the patent: **03.07.2019**
(21) Application number: 16176663.9
(22) Date of filing: 25.09.2007
(51) Int. Cl.: A61K 31/135, A61K 47/38, A61P 37/06, A61K 9/20, A61K 9/48, A61K 9/50, A61K 9/28, A61K 9/16

(54) **PHARMACEUTICAL COMPOSITIONS COMPRISING AN S1P MODULATOR**
PHARMAZEUTISCHE ZUSAMMENSETZUNGEN MIT EINEM S1P-MODULATOR
COMPOSES PHARMACEUTIQUES COMPRENANT UN MODULATEUR S1P

(30) Priority: 26.09.2006 EP 06121299; 26.09.2006 EP 06121301; 26.09.2006 EP 06121302; 26.09.2006 EP 06121276
(43) Date of publication of application: 14.12.2016
(62) Divisional of application: 07818399.3
(73) Proprietor: NOVARTIS AG, 4056 Basel (CH)
(72) Inventor: AMBÜHL, Michael, 4123 Allschwil (CH); BEYER, Jutta, 4056 Basel (CH); CARRENO-GOMEZ, Begona, 4056 Basel (CH); RUEGGER, Colleen, Morris Plains, NJ 07950 (US); VALAZZA, Stephen, Matawan, NJ 07747 (US)
(74) Representative: Wiessner, Michael

(56) References cited:
- EP-A1- 1 195 165
- EP-A1- 1 201 236
- EP-A2- 1 661 881
- WO-A-2004/089341
- WO-A2-2005/002559
- WO-A2-2005/058295
- CN-A- 1 524 518
- BRINKMANN V ET AL: "FTY720: targeting G-protein-coupled receptors for sphingosine 1-phosphate in transplantation and autoimmunity", CURRENT OPINION IN IMMUNOLOGY, CURRENT BIOLOGY LTD, vol. 14, no. 5, 1 October 2002 (2002-10-01), pages 569-575, XP004377409, ISSN: 0952-7915
- Raymond C Rowe ET AL: "Handbook of Pharmaceutical Excipients, Sixth edition /Passage/" In: "Handbook of Pharmaceutical Excipients", 1 January 2009 (2009-01-01), Pharmaceutical Press, XP055397596, ISBN: 978-0-85369-792-3 pages 3pp,222, 364, 424, 425, 679-703,
- KIBBE H (EDITOR) ED - KIBBE A H (ED): "HANDBOOK OF PHARMACEUTICAL EXCIPIENTS, CELLULOSE, MICROCRYSTALLINE", HANDBOOK OF PHARMACEUTICAL EXCIPIENTS, AMERICAN PHARMACEUTICAL ASSOC. [U.A.], WASHINGTON, DC; US, 1 January 2000 (2000-01-01), pages 102-103, XP001544055, ISBN: 978-0-85369-381-9
- STEPHEN R BYRN ET AL: "Chemical reactivity in solid-state pharmaceuticals: formulation implications", ADVANCED DRUG DELIVERY REVIEWS, ELSEVIER, AMSTERDAM, NL, vol. 48, no. 1, 16 May 2001 (2001-05-16), pages 115-136, XP002628347, ISSN: 0169-409X, DOI: 10.1016/S0169-409X(01)00102-8 [retrieved on 2001-04-20]
- "Chapter 6: Hilfsstoffe; Section 3.1: Zucker und Zuckeralkohole" In: Bauer, Kurt H.; Frömming, Karl-Heinz; Führer, Claus: "Pharmazeutische Technologie", 1993, Georg Thieme verlag Stuttgart ISBN: 3136925041 page 160,

## Description

### Organic Compounds

The present disclosure, with the invention as defined in the independent claims and in preferred form in the dependent claims, relates to pharmaceutical compositions comprising a sphingosine-1 phosphate receptor modulator, in particular a sphingosine-1 phosphate receptor agonist.

Sphingosine-1 phosphate (hereinafter referred to as "S1P") is a natural serum lipid. Presently there are 8 known S1P receptors, namely S1P1 to S1P8. S1P receptor agonists have accelerating lymphocyte homing properties.

S1P receptor agonists are immunomodulating compounds which elicit a lymphopenia resulting from a re-distribution, preferably reversible, of lymphocytes from circulation to secondary lymphatic tissue, evoking a generalized immunosuppression. Naive cells are sequestered, CD4 and CD8 T-cells and B-cells from the blood are stimulated to migrate into lymph nodes (LN) and Peyer's patches (PP), and thus infiltration of cells into transplanted organs is inhibited.

WO 2004/089341 describes compositions comprising an S1P receptor agonist such as 2-amino-2-[2-(4-octylphenyl)ethyl]propane-1,3-diol and an obligatory carrier in the form of a sugar alcohol, such as mannitol, allowing promoting uniform distribution of the S1P receptor agonist and improving compressibility and hardnes of tablets formed from the composition.

EP 1 201 236 describes compositions for preventing or treating viral myocarditis containing 2-amino-2-[2-(4-octylphenyl)ethyl]propane-1,3-diol and, without raising any concerns regarding compatibility, mentions a tablet composition consisting of that compound, lactose, magnesium stearate and about 21 weight % "crystalline cellulose".

CN1524518 A discloses compositions for preventing or treating liver injury comprising 2-amino-2[2-(4-octylphenyl)ethyl]propane-1,3-diol or a salt thereof, and mentions solid compositions comprising such a compound formulated with 33% microcrystalline cellulose as well as lactose, starch, and magnesium stearate as additional excipients.

The various known S1P receptor modulators show structural similarities, which result in related problems in providing a suitable formulation. There exists a need for an S1P receptor modulator containing formulation which is well-adapted for oral administration in a solid form, e.g. as a tablet or capsule. In addition, the oral route is often the most convenient route for drug administration, but unfortunately many patients have difficulties in swallowing, e.g. due to an unpleasant taste of the dosage form or there being no water available at the time of ingestion. Thus, there also exists a need for an S1P receptor modulator containing oral formulation which can easily be swallowed, e.g. by children or older patients. Furthermore, there is a need for a way in which to readily produce dosage forms of S1P receptor modulators having a variety of dosage strengths.

The present disclosure provides various pharmaceutical compositions containing an S1P receptor modulator which address these needs. The compositions provide a convenient means of systemic administration of S1P receptor agonists and other modulators, do not suffer from the disadvantages of liquid formulations for injection or oral use, and have good physicochemical and storage properties. In particular, the compositions of the present invention may show a high level of uniformity in the distribution of the S1P receptor modulator throughout the composition, as well as high stability. The compositions of the invention as defined in the claims may be manufactured on high speed automated equipment, and thus do not require hand encapsulation.

The pharmaceutical compositions of the present invention may be produced by standard processes, for instance by conventional mixing, granulating, dissolving or lyophilizing processes. Procedures which may be used are known in the art, e.g. those described in L. Lachman et al. The Theory and Practice of Industrial Pharmacy, 3rd Ed, 1986, H. Sucker et al, Pharmazeutische Technologie, Thieme, 1991, Hagers Handbuch der pharmazeutischen Praxis, 4th Ed. (Springer Verlag, 1971) and Remington's Pharmaceutical Sciences, 13th Ed., (Mack Publ., Co., 1970) or later editions.

The compositions of the invention may show good stability characteristics as indicated by standard stability trials, for example having a shelf life stability of up to one, two or three years, and even longer. Preferably, the compositions are stable for at least six months at ambient temperature. Stability characteristics may be determined, e.g. by measuring decomposition products by HPLC analysis after storage for particular times, at particular temperatures, e.g. 20°, 40° or 60 °C.

### Compositions In Which Sugar Alcohol Is Absent

The invention provides a solid pharmaceutical composition suitable for oral administration, comprising:
(a) a S1P receptor modulator, e.g. an S1P agonist; and
(b) 75 to 99.99 % by weight of a microcrystalline cellulose
in the absence of a sugar alcohol,
wherein the S1P receptor modulator is 2-amino-2-[2-(4-octylphenyl)ethyl]propane-1,3-diol in free form or in a pharmaceutically acceptable salt form.

The composition may further comprise a lubricant.

Suitable lubricants include stearic acid, magnesium stearate, calcium stearate, zinc stearate, glyceryl palmitostearate, sodium stearyl fumarate, canola oil, hydrogenated vegetable oil such as hydrogenated castor oil (e.g. Cutina® or Lubriwax® 101), mineral oil, sodium lauryl sulfate, magnesium oxide, colloidal silicon dioxide, polyethylene glycol, polyvinyl alcohol, sodium benzoate, talc, poloxamer, or a mixture of any of the above.

Preferably the lubricant comprises magnesium stearate or a hydrogenated vegetable oil.

The composition preferably contains 0.01 to 5% by weight of the lubricant, more preferably 1 to 3% by weight, e.g. about 2% by weight, based on the total weight of the composition.

The composition may comprise one or more further excipients such as carriers, binders or diluents.

The composition may comprise an additional binder for example, methylcellulose, hydroxypropylcellulose, hydroxypropylmethylcellulose, dicalcium phosphate, PVP, e.g. cellulose, polyethylene glycols, polyvinylpyrrolidone, starch mucilage, acacia, alginic acid, carboxymethylcellulose, hydroxyethylcellulose,, dextrin, ethylcellulose, gelatin, guar gum, hydroxypropylmethylcellulose, magnesium aluminium silicate, kaltodectrin, methylcellulose, polyethylene oxide, povidone, sodium alginate or hydrogenated vegetable oils.

The composition may also include, or alternatively include, glidants, e.g. silica.

The composition may be in form of a powder, granule or pellets or in unit dosage form, for example as a tablet or capsule. The compositions are well-adapted for encapsulation into an orally administrable capsule shell, particularly a hard gelatin shell. Alternatively the compositions may be compacted into tablets.

Tablets may be coated, for instance with talc or a polysaccharide (e.g. cellulose) or hydroxypropylmethylcellulose coating.

The composition may also additionally comprise disintegrants. Examples of disintegrants are, for example, crosscarmellose cellulose, crosspovidone and sodium starch glycolate.

The composition may also include, or alternatively include, surfactants, e.g. sodium lauryl sulphate or docusate sodium.

The composition may also include, or alternatively include, gas producers, e.g. sodium bicarbonate or citric acid.

The composition may comprise a release rate controlling additive. For example, the drug may be held within a hydrophobic polymer matrix so that it is gradually leached out of the matrix upon contact with body fluids.

Alternatively, the drug may be held within a hydrophilic matrix which gradually or rapidly dissolves in the presence of body fluid. The tablet core may comprise two or more layers having different release properties. The layers may be hydrophilic, hydrophobic or a mixture of hydrophilic and hydrophobic layers. Adjacent layers in a multilayer tablet core may be separated by an insoluble barrier layer or hydrophilic separation layer. An insoluble barrier layer may be formed of materials used to form the insoluble casing. A hydrophilic separation layer may be formed from a material more soluble than the other layers of the tablet core so that as the separation layer dissolves the release layers of the tablet core are exposed.

Suitable release rate controlling polymers include polymethacrylates, ethylcellulose, hydroxypropylmethylcellulose, methylcellulose, hydroxyethylcellulose, hydroxypropylcellulose, sodium carboxymethylcellulose, calcium carboxymethylcellulose, acrylic acid polymer, polyethylene glycol, polyethylene oxide, carrageenan, cellulose acetate, zein etc.

The composition may additionally include materials which swell on contact with aqueous liquids, and which may be included in the composition, include polymer materials selected from cross-linked sodium carboxymethylcellulose, cross-linked hydroxypropylcellulose, high molecular weighthydroxypropylcellulose, carboxymethylamide, potassium methacrylatedivinylbenzene copolymer, polymethylmethacrylate, cross-linked polyvinylpyrrolidone and high molecular weight polyvinylalcohols.

In one embodiment, the composition includes a silicon dioxide.

The microcrystalline cellulose may act as a diluent, carrier, filler or bulking agent, and may suitably be Avicel®. The size of the particles of the microcrystalline cellulose may vary.

The use of microcrystalline cellulose composition may assist in promoting uniform distribution of the S1P receptor modulator throughout the microcrystalline cellulose in the composition. A higher surface area may be achieved by providing a microcrystalline cellulose preparation consisting of particles having a smaller mean size and/or a rougher surface on each particle.

The use of micronized microcrystalline cellulose, e.g. with a mean particle size of 30 µm or less, has also been found to improve compressibility and hardness of tablets formed from the composition.

The composition contains 75 to 99.99% by weight of the microcrystalline cellulose, e.g. 85 to 99.9%, e.g. 90 to 99.5% by weight, based on the total weight of the composition.

Typically sugar alcohols include mannitol or sorbitol.

Compositions of the present invention may be in the form of, for example, tablets, capsules, caplets, lozenges, pills, mini-tablets, pellets, beads or granules.

Where the solid composition is in the form of pellets or granules, these may, after application of the coating as described hereinafter, be used as such or to fill capsules, e.g. hard gelatine capsules or other storage means, for example sachets prior to administration.

Pellets and granules may be from 2 to 0.3 mm in diameter, for example, a "normal pellet" has a size of 1 to 0.6 mm and a "bead pellet" has a size of 0.4 to 0.8 mm

The present invention also provides a process for producing a pharmaceutical composition, comprising:
(a) mixing an S1P receptor agonist or other modulator with a microcrystalline cellulose in an amount as given above, e.g. Avicel®,;
(b) milling and/or granulating the mixture obtained in (a); and
(c) optionally mixing the milled mixture obtained in (b) with a lubricant.

By using this process, a preparation having a good level of content and blend uniformity (i.e. a substantially uniform distribution of the S1P receptor modulator throughout the composition), dissolution time and stability is obtained.

The S1P receptor modulator, 2-amino-2-[2-(4-octylphenyl)ethyl]propane-1,3-diol, hydrochloride, may optionally be micronized, and/or pre-screened, e.g. with a 400 to 500 µm mesh screen, before step (a) in order to remove lumps. The mixing step (a) may suitably comprise blending the S1P receptor agonist and the microcrystalline cellulose, e.g. Avicel®, in any suitable blender or mixer for e.g. 100 to 400 revolutions.

The process may be carried out by dry mixing the components. In this case, the milling step (b) may suitably comprise passing the mixture obtained in (a) through a screen, which preferably has a mesh size of 400 to 500 µm. Process step (a) may comprise the step of mixing the total amount of S1P receptor agonist at first with a low amount of microcrystalline cellulose, e.g. Avicel®, e.g. from 5 to 25% by weight of the total weight of microcrystalline cellulose, e.g. Avicel®, in order to form a pre-mix. Subsequently the remaining amount of microcrystalline cellulose, e.g. Avicel®, is added to the pre-mix. Step (a) may also comprise the step of adding a binder solution, e.g. methylcellulose and/or xylitol, e.g. an aqueous solution, to the mixture.

The milled mixture obtained in (b) may optionally be blended once more before mixing with the lubricant. The lubricant, e.g. magnesium stearate, is preferably pre-screened, e.g. with a 800 to 900 µm screen, before mixing.

Alternatively, a wet granulation process is employed. In this embodiment, the S1P receptor modulator is preferably first dry-mixed with the desired microcrystalline cellulose, e.g. Avicel®, and the obtained microcrystalline cellulose, e.g. Avicel®/S1P receptor modulator mixture is then dry-mixed with a binder such as hydroxypropyl cellulose or hydroxypropylmethyl cellulose. Water is then added and the mixture granulated, e.g. using an automated granulator. The granulation is then dried and milled.

If desirable, an additional amount of binder may be added in step (c) to the mixture obtained in (b).

The process may comprise a further step of tabletting or encapsulating the mixture obtained in (c), e.g. into a hard gelatin capsule using an automated encapsulation device. The capsules may be coloured or marked so as to impart an individual appearance and to make them instantly recognizable. The use of dyes can serve to enhance the appearance as well as to identify the capsules. Dyes suitable for use in pharmacy typically include carotinoids, iron oxides, and chlorophyll. Preferably, the capsules are marked using a code.

### S1P Modulators

The S1P receptor agonist is 2-amino-2-[2-(4-octylphenylethyl)]propane-1,3-diol in free form or in a pharmaceutically acceptable salt form (referred to hereinafter as Compound A), e.g. the hydrochloride, i.e. FTY720, as shown:

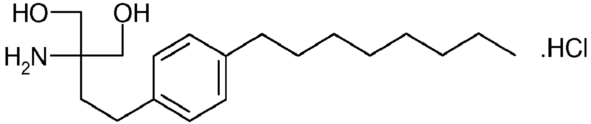

Examples of pharmaceutically acceptable salts include salts with inorganic acids, such as hydrochloride, hydrobromide and sulfate, salts with organic acids, such as acetate, fumarate, maleate, benzoate, citrate, maleate, methanesulfonate and benzenesulfonate salts, or, when appropriate, salts with metals, such as sodium, potassium, calcium and aluminium, salts with amines, such as triethylamine and salts with dibasic amino acids, such as lysine. The compounds and salts of the present invention encompass hydrate and solvate forms.

The composition of the present invention may comprise one or more salts and/or free acid of the S1P modulator.

The composition of the invention preferably contains 0.01 to 20% by weight of S1P receptor modulator, more preferably 0.1 to 10%, e.g. 0.5 to 5% by weight, based on the total weight of the composition.

Where the pharmaceutical capsule is in unit dosage form, each unit dosage may suitably contain 0.5 to 10 mg of the S1P receptor modulator.

### Use

Pharmaceutical compositions of the present invention are useful, either alone or in combination with other active agents, for the treatment and prevention of conditions e.g. as disclosed in US 5,604,229, WO 97/24112, WO 01/01978, US 6,004,565, US 6,274,629 and JP-14316985.

The compositions described herein may promote the absorption and distribution of the S1P modulator through the blood brain barrier and into the brain.

In particular, the pharmaceutical compositions are useful for:
a) treatment and prevention of organ or tissue transplant rejection, for example for the treatment of the recipients of heart, lung, combined heart-lung, liver, kidney, pancreatic, skin or corneal transplants, and the prevention of graft-versus-host disease, such as sometimes occurs following bone marrow transplantation; particularly in the treatment of acute or chronic allo- and xenograft rejection or in the transplantation of insulin producing cells, e.g. pancreatic islet cells;
b) treatment and prevention of autoimmune disease or of inflammatory conditions, e.g. multiple sclerosis, arthritis (for example rheumatoid arthritis), inflammatory bowel disease, hepatitis, etc.;
c) treatment and prevention of viral myocarditis and viral diseases caused by viral mycocarditis, including hepatitis and AIDS.

The invention is, in one embodiment, related to the treatment of inflammatory conditions. In one example, the invention is related to compositions for the control and/or suppression of mast cell activation and secretion for the relief of inflammatory conditions, e.g. in the brain as in multiple sclerosis.

There is also provided, for reference disclosure purposes, a method of protecting multiple sclerosis subjects against neurodegerative brain inflammation, comprising the administration to said subjects a composition as described herein, for example a composition comprising an S1P agonist or other modulator.

Compositions of the present invention may be for use in administration in an amount which is therapeutically effective against a disease or condition which can be treated by administration of the S1P receptor modulator.

The exact amount of S1P receptor modulator or pharmaceutically acceptable salt thereof to administer can vary widely. The dose may depend on the particular compound, route of administration, the rate of administration, the nature of the disease or condition being treated, and the sex, age and body weight of the patient. The dose may also depend on the existence, nature and extent of any adverse side-effects that may accompany the administration of the concentrate or pharmaceutical formulation. Typically, a dose of 0.5 to 5 mg of S1P receptor modulator Compound A, are administered to children.

The composition of the present invention and any concentrate for dilution and respective pharmaceutical solution may be for use in combination with other immunosuppressant(s), steroid(s) such as prednisolone, methylprednisolone, dexamethasone, hydrocortisone and the like, or nonsteroidal anti-inflammatory agent. The administration of a combination of active agents may be simultaneous or consecutive, with either one of the active agents being administered first. The dosage of the active agents of a combination treatment may depend on effectiveness and site of action of each active agent, as well as synergistic effects between the agents for use for combination therapy.

The invention especially concerns the following embodiments:
(A) A solid pharmaceutical composition suitable for oral administration, comprising:
   (a) an S1P receptor modulator; and
   (b) 75 to 99.99 % by weight of a microcrystalline cellulose
   in the absence of a sugar alcohol,
   wherein the S1P receptor modulator is 2-amino-2-[2-(4-octylphenyl)ethyl]propane-1,3-diol in free form or in a pharmaceutically acceptable salt form.
(B) A composition according to paragraph (A), comprising 85 to 99.9 % by weight of microcrystalline cellulose.3. A composition according to claim 1, comprising 90 to 99.5% by weight of the microcrystalline cellulose.
(C) A composition according to paragraph (A), comprising 0.5 to 5 % by weight of 2-amino-2-[2-(4-octylphenyl)ethyl]propane-1,3-diol, or a pharmaceutically acceptable salt thereof.
(D) A composition according to any one of the preceding paragraphs (A) to (C) wherein the S1P receptor modulator is 2-amino-2-[2-(4-octylphenyl)ethyl]propane-1,3-diol hydrochloride.
(E) A composition according to paragraph (A) in the form of tablets, capsules, caplets, lozenges, pills, mini-tablets, pellets, beads or granules.
(F) A composition according to paragraph (A) in the form of a powder, a granule or pellets.
(G) A composition according to paragraph (A) in the form of a tablet.
(H) A composition according to paragraph (A) in the form of a capsule.
(I) A process for producing a pharmaceutical composition of paragraph (A), comprising the steps:
   (a) mixing the S1P receptor modulator with the microcrystalline cellulose;
   (b) milling the mixture obtained in (a); and
   (c) mixing the milled mixture obtained in (b) with a lubricant.

The invention will now be described with reference to the following specific Examples.

### Reference-Example 1

Micronized Compound 2-amino-2-[2-(4-octylphenyl)ethyl]propane-1,3-diol, hydrochloride salt (FTY720), is screened mixed with the microcrystalline cellulose agent, e.g. Avicel PH 102. The mixture is then milled in a Frewitt MGI device (Key International Inc. USA) using a 30 mesh screen. Magnesium stearate is screened using a 20 mesh screen and blended with the FTY720/cellulosemixture. Crosscarmellose is the blended to produce a product composition.

An example for a 6 mm round, 80 mg tablet core obtained by direct compression is shown below:

| Ingredient | mg/dose |
|---|---|
| FTY720 HCl | 1.40 |
| Microcrystalline cellulose, e.g. Avicel PH 102 | 73.80 |
| Magnesium stearate | 0.80 |
| Crosscarmellose | 4.00 |

As an alternative, a core tablet composition may be compacted on a tablet press using a 7 mm die to form 120 mg tablets, an example of which may be:

| Ingredient | mg/dose |
|---|---|
| FTY720 HCl | 1.40 |
| Mannitol M200 | 116.20 |
| Magnesium stearate | 2.40 |

1 mg of FTY720 in free form is equivalent to 1.12 mg of FTY720 HCI salt.

### Reference-Example 2

In a further example, the process of Example 1 is repeated except that the magnesium stearate is replaced by Cutina® (hydrogenated castor oil).

### Reference-Example 3

In a further Example, the tablets are prepared as described in Examples 1 and 2, except that FTY720 is replaced in each case by 2-amino-2-{2-[4-(1-oxo-5-phenylpentyl)phenyl]ethyl} propane-1,3-diol hydrochloride.

### Reference-Examples 4 to 7

Tablets containing the following ingredients (in mg) are produced:

| | Example 4 | Example 5 | Example 6 | Example 7 |
|---|---|---|---|---|
| FTY720 | 1 | 1 | 1 | 1 |
| D-mannitol | 62.3 | 62.3 | 62.0 | 62.0 |
| Xylitol* | 26.7(5.4) | 26.7(5.4) | 26.6 | 26.6 |
| Methylcellulose | - | - | 0.4 | 0.4 |
| Microcrystalline Cellulose | 24.0 | - | 24.0 | - |
| Low-substituted HydroxypropylCellulose | - | 24.0 | - | 24.0 |
| Hydrogenated oil | 6.0 | 6.0 | 6.0 | 6.0 |
| Total | 120.0 | 120.0 | 120.0 | 120.0 |

| | | | | |
|---|---|---|---|---|
| * The amount of xylitol indicated in brackets was used as a binder. | | | | |

FTY720, D-mannitol and xylitol are placed in a fluid-bed granulator (MP-01 model, Powrex), mixed for five minutes, and granulated under spray of binder solution, followed by drying till the exhaust temperature reaches 40 °C. The granulation conditions are as shown below. Dried powder is passed through a 24-mesh sieve, added to the specified amount of filler and lubricant, and mixed in a mixer (Tubular Mixer, WAB) for three minutes to make the powder for compression.

The resulting powder is compressed by a tabletting machine (Cleanpress correct 12 HUK, Kikushui Seisakusho) with a punch of 7 mm i.d. x 7.5 mm R at a compression force of 9800 N.

**Granulation conditions:**

| Item | Setting |
|---|---|
| Charge-in amount | 1170 g |
| Volume of intake-air | 50 m³/min |
| Temperature of intake-air | 75 °C |
| Flow rate of spray solution | 15 mL/min |
| Spray air pressure | 15 N/cm² |
| Spray air volume | 30 L/min |
| Volume of binder solution | 351 mL |

### Reference-Example 8

An example powder coating composition:
The components are premixed under high shear, then wet granulated by mixing under high shear with water. The granulated mixture is dried in fluid bed drier to reduce the moisture content to below 3% by weight. The dried granules are milled and micronised to a powder.

| Ingredient | Composition (% w/w) |
|---|---|
| Ammonio-methacrylate co-polymer, e.g. Eudragit RS | 46.5 |
| hydroxy propyl cellulose, e.g. Klucel | 28.0 |
| titanium dioxide | 15.0 |
| aluminium lake | 5.0 |
| polyethylene glycol 6000 | 5.0 |
| colloidal silicon dioxide, e.g. Aerosil 200 | 0.5 |

### Reference-Example 9

An example powder coating composition:

| Ingredient | Composition (% w/w) |
|---|---|
| Ammonio-methacrylate co-polymer, e.g. Eudragit RS | 39.75 |
| hydroxy propyl cellulose, e.g. Klucel | 39.75 |
| titanium dioxide | 15.0 |
| aluminium lake | 5.0 |
| colloidal silicon dioxide, e.g. Aerosil | 0.5 |

### Reference-Example 10

An example liquid coating composition (aqueous dispersion):
At the fusing or drying stations, energy is imparted to the core surfaces to fuse the powder or dry the liquid and provide a uniform coating on the exposed surfaces of the core. The energy is provided by focused radiation preferably in the infra-red region; the energy power requirement will be determined largely by the coating material. After fusing or drying, the coating is set by cooling, using an air blower.

| Ingredient | Composition (% w/w) |
|---|---|
| hydroxypropylmethylcellulose | 70 |
| Glycerol | 7 |
| iron oxide yellow | 23 |

### Reference-Example 11

An example for a 7 mm round, 127 mg tablet for fast disintegration according to the present invention:

| Ingredient | mg/dose |
|---|---|
| FTY720 HCl | 0.56 |
| Directly compressible mannitol, e.g. Parteck M200 | 82.54 |
| Calcium silicate | 36.00 |
| Magnesium stearate | 0.90 |
| Crospovidone | 7.00 |

The tablet may be manufactured by known methods. For example, the tablet may be manufactured by blending of all ingredients and further compressing to tablets and/or granulation and/or micronisation and further compressing of the granules to tablets.

### Reference-Example 12

A rapid disintegrating formulation is prepared, which comprises gelatin (3%), mannitol as structure forming agent (1-5%), sweeteners, flavoring agents.

Gelatin and mannitol are added to the water and heated to 40°C to dissolve. The gelatin/mannitol solution is cooled to 23 °C and mixed with the active ingredient, e.g. an S1P agonist or other modulator. The total solid content is less than 50%. The suspension is first cooled to 15 °C to prevent sedimentation of the suspension before the start of the lyophilization (coated or uncoated).

### Reference-Example 13

As Example 12, except where the mannitol is replaced with sorbitol.

### Example 1

Micronized compound 2-amino-2-[2-(4-octylphenyl)ethyl]propane-1,3-diol, hydrochloride salt (FTY720), is screened mixed with the microcrystalline cellulose agent, e.g. Avicel PH 102. The mixture is then milled in a Frewitt MGI device (Key International Inc. USA) using a 30 mesh screen. Magnesium stearate is screened using a 20 mesh screen and blended with the FTY720/cellulosemixture. Crosscarmellose is the blended to produce a product composition.

An example for a 6 mm round, 80 mg tablet core obtained by direct compression is shown below:

| Ingredient | mg/dose |
|---|---|
| FTY720 HCl | 1.40 |
| Microcrystalline cellulose, e.g. Avicel PH 102 | 73.80 |
| Magnesium stearate | 0.80 |
| Crosscarmellose | 4.00 |

### Example 2

Micronized Compound A, e.g. 2-amino-2-[2-(4-octylphenyl)ethyl]propane-1,3-diol, hydrochloride salt (FTY720), is screened and 116.7 g of the screened compound is mixed with 9683.3 g of a microcrystalline cellulose agent. The mixture is then milled in a Frewitt MGI device (Key International Inc. USA) using a 30 mesh screen. Magnesium stearate is screened using a 20 mesh screen and 200 g of the screened compound blended with the FTY720 mixture to produce a product composition.

The product composition is then compacted on a tablet press using a 7 mm die to form 120 mg tablets, each containing:

| | |
|---|---|
| Compound A, e.g. FTY720 * | 1.4 mg |
| Microcrystalline cellulose, e.g. Avicel PH 102 | 116.2 mg |
| Magnesium stearate | 2.4 mg |
| Total | 120 mg |

| | |
|---|---|
| * 1 mg of Compound A in free form is equivalent to 1.12 mg of FTY720. | |

### Example 3

In a further example, the process of Example 2 is repeated except that the magnesium stearate is replaced by Cutina® (hydrogenated castor oil).

### Example 4

FTY720, and microcrystalline cellulose, e.g. Avicel PH 102 are each screened separately using an 18 mesh screen. 1.9 g screened FTY720 is mixed with 40 g screened microcrystalline cellulose agent for 120 revolutions in a blender at 32 rpm. The FTY720 mixture is then screened through a 35 mesh screen.

The screened FTY720 mixture is added to a granulator along with a further 340.1 g Microcrystalline cellulose, e.g. Avicel PH 102 and 12 g hydroxypropylcellulose. The mixture is mixed for 3 minutes. Water is then added at a rate of 100 ml/minute and the mixture granulated for 2 minutes. The granulation is transferred into a tray dryer and dried at 50 °C for 150 minutes.

The mixture is then milled in a Frewitt MGI device using a 35 mesh screen. Magnesium stearate is screened and 6 g of the screened compound is blended for 90 revolutions at 32 rpm with the FTY720 mixture to produce a product composition showing a substantially uniform distribution of the S1P receptor agonist throughout the microcrystalline cellulose, e.g. Avicel PH 102 in the blend.

The product composition is then filled into size 3 hard gelatin shells on an H & K 400 encapsulation device. 120 mg of the product composition is added to each capsule. Therefore each capsule contains:

| | |
|---|---|
| FTY720 * | 0.56 mg |
| Microcrystalline cellulose | 114.04 mg |
| Hydroxypropylcellulose | 3.6 mg |
| Magnesium stearate | 1.8 mg |
| | |
| Total | 120 mg |

### Example 5

In a further example, the process of Example 4 is repeated except that the magnesium stearate is replaced by Cutina® (hydrogenated castor oil).

### Example 6

In a further example, the process of Example 4 is repeated except that the hydroxypropyl cellulose is replaced by hydroxypropylmethyl cellulose.

### Example 7

Micronized FTY720, is screened using a 425 µm (40 mesh) screen. 58.35 g of the screened compound is mixed with 4841.65 g microcrystalline cellulose, e.g. Avicel PH 102 in a 25L Bohle bin blender for 240 blending revolutions. The mixture is then milled in a Frewitt MGI device using a 425 µm mesh screen, and the milled mixture is blended once more. Magnesium stearate is screened and 100 g of the screened compound is blended with the FTY720 mixture to produce a product composition showing a substantially uniform distribution of the S1P receptor agonist throughout the blend.

The product composition is then filled into size 3 hard gelatin shells on an H & K 400 encapsulation device. 120 mg of the product composition is added to each capsule. Therefore each capsule contains:

| | |
|---|---|
| FTY720 * | 1.4 mg |
| Microcrystalline cellulose | 116.2 mg |
| Magnesium stearate | 2.4 mg |
| | |
| Total | 120 mg |

### Examples 8 and 9

In further examples, capsules are prepared as described in Example 7, except that each capsule contains each component in the following amounts:

| | Example 8 | Example 9 |
|---|---|---|
| FTY720 * | 2.8 mg | 5.6 mg |
| Microcrystalline cellulose | 114.8 mg | 112 mg |
| Magnesium stearate | 2.4 mg | 2.4 mg |
| | | |
| Total | 120 mg | 120 mg |

### Examples 10 to 12

In further examples, capsules are prepared as described in Examples 7 to 9, except that the magnesium stearate is replaced in each case by Cutina® (hydrogenated castor oil).

### Reference-Examples 14 to 24

In further examples, capsules or tablets are prepared as described in Examples 1 to 11, except that FTY720 is replaced in each case by 2-amino-2-{2-[4-(1-oxo-5-phenylpentyl)phenyl]ethyl}propane-1,3-diol hydrochloride.

### Examples 13 to 15

Pharmaceutical compositions containing the following ingredients are produced:

| | Example 13 | Example 14 | Example 15 |
|---|---|---|---|
| FTY720 | 5 g | 10 g | 100 g |
| Microcrystalline cellulose | 991 g | 986 g | 897 g |
| Methylcellulose SM-25 | 4 g | 4 g | 3 g |
| | | | |
| Total | 1000 g | 1000 g | 1000 g |

The FTY720 and a proportion of the microcrystalline cellulose, e.g. Avicel PH 102 equal to twice the weight of the FTY720 are mixed in a Microspeed Mixer MS-5 type (Palmer, USA) for 2 minutes at 1200 rpm. The remaining microcrystalline cellulose is added to the mixture and mixed for another 2 minutes. 80 or 60 milliliters of 5% methylcellulose SM-25 solution is supplied from a hopper and granulated under the same conditions. The mixture is extruded through a screen with 0.4 mm apertures using an extruder RG-5 type. The extruded material is dried at 65°C by a fluidized-bed granulator STREA I Type (Patheon, Canada) and then sieved through a 24 mesh sieve. Fine particles which pass through a 60 mesh sieve are removed. The obtained fine granules are filled into capsules by a Zuma capsule-filling machine (100 mg per capsule).

### Example 16 and Reference-Example 25

An example of a tablet formulation comprising 1.25 mg FTY720 obtainable by wet granulation.

**Composition for wet granulation (Reference-Example 25):**

| Ingredient | mg/tablet | % |
|---|---|---|
| FTY HCI | 1.49 | 1.49 |
| HPMC 3cps | 3.00 | 3.00 |
| Water granulation liquid | q.s | q.s |
| Mannitol | 46.25 | 46.25 |
| Avicel PH 101 | 46.25 | 46.25 |
| Aerosil 200 | 3.01 | 3.01 |
| Croscarmellose | 5.00 | 5.00 |
| Magnesium stearate | 1.00 | 1.00 |
| Total | 100.00 | 100.00 |

Microcrystalline cellulose was wet granulated with an aqueous solution of FTY720 and HPMC. After drying, the mixture was sieved and blended with mannitol, silicon dioxide, croscarmellose and magnesium stearate, and compressed into 6 mm round tablets of 100 mg.

This formulation can alternatively be manufactured without sugar alcohols such as mannitol, using microcrystalline cellulose instead (Example 16):

| Ingredient | mg/tablet | % |
|---|---|---|
| FTY HCI | 1.49 | 1.49 |
| HPMC 3cps | 3.00 | 3.00 |
| Water granulation liquid | q.s | q.s |
| Avicel PH 101 | 92.50 | 92.50 |
| Aerosil 200 | 3.01 | 3.01 |
| Croscarmellose | 5.00 | 5.00 |
| Magnesium stearate | 1.00 | 1.00 |
| Total | 100.00 | 100.00 |

### Reference-Example 26

An example of coating composition comprising FTY720.

**Composition for coating of pellets, minitablets and small tablets**

| Ingredient | mg/tablet | % |
|---|---|---|
| HPMC 3cps | 1.62 | 11.60 |
| FTY HCl | 0.04 | 0.25 |
| Butylhydroxytoluol | 0.01 | 0.05 |
| Triethylcitrate | 0.07 | 0.50 |
| Acetone | 6.12 | 43.81 |
| Ethanol | 6.12 | 43.81 |
| Total Dry | 1.74 | 12.39 |
| Total | 100.00 | 100.00 |

The polymer HPMC can also be replaced by, for example, HPC or other comparable polymers. The FTY720 coat can be applied to active or placebo pellets, minitablets or small tablets separated by, for example, a protection coat (e.g. HPMC) and/or covered with an overcoat (e.g. HPMC). This dosage form can be filled into capsules (e.g. HPMC or HGC) or stickpacks and hence is flexible in the sense that different dosage strengths or combination products may be formulated.

## Claims

1. A solid pharmaceutical composition suitable for oral administration, comprising:
(a) an S1P receptor modulator; and
(b) 75 to 99.99 % by weight of a microcrystalline cellulose
in the absence of a sugar alcohol,
wherein the S1P receptor modulator is 2-amino-2-[2-(4-octylphenyl)ethyl]propane-1,3-diol in free form or in a pharmaceutically acceptable salt form.

2. A composition according to claim 1, comprising 85 to 99.9 % by weight of microcrystalline cellulose.

3. A composition according to claim 1, comprising 90 to 99.5% by weight of the microcrystalline cellulose.

4. A composition according to claim 1, comprising 0.5 to 5 % by weight of 2-amino-2-[2-(4-octylphenyl)ethyl]propane-1,3-diol, or a pharmaceutically acceptable salt thereof.

5. A composition according to any one of the preceding claims wherein the S1P receptor modulator is 2-amino-2-[2-(4-octylphenyl)ethyl]propane-1,3-diol hydrochloride.

6. A composition according to claim 1 in the form of tablets, capsules, caplets, lozenges, pills, mini-tablets, pellets, beads or granules.

7. A composition according to claim 1 in the form of a powder, a granule or pellets.

8. A composition according to claim 1 in the form of a tablet.

9. A composition according to claim 1 in the form of a capsule.

10. A process for producing a pharmaceutical composition of claim 1, comprising the steps:
(a) mixing the S1P receptor modulator with the microcrystalline cellulose;
(b) milling the mixture obtained in (a); and
(c) mixing the milled mixture obtained in (b) with a lubricant.

## Patentansprüche

1. Für die orale Verabreichung geeignete feste pharmazeutische Zusammensetzung, umfassend:
(a) einen Modulator des S1P-Rezeptors und
(b) 75 bis 99,99 Gew.-% einer mikrokristallinen Cellulose
in Abwesenheit eines Zuckeralkohols,
wobei es sich bei dem Modulator des S1P-Rezeptors um 2-Amino-2-[2-(4-octylphenyl)ethyl]propan-1,3-diol in freier Form oder in Form eines pharmazeutisch unbedenklichen Salzes handelt.

2. Zusammensetzung nach Anspruch 1, die 85 bis 99,9 Gew.-% mikrokristalline Cellulose umfasst.

3. Zusammensetzung nach Anspruch 1, die 90 bis 99,5 Gew.-% der mikrokristallinen Cellulose umfasst.

4. Zusammensetzung nach Anspruch 1, die 0,5 bis 5 Gew.-% 2-Amino-2-[2-(4-octylphenyl)ethyl]propan-1,3-diol oder eines pharmazeutisch unbedenklichen Salzes davon umfasst.

5. Zusammensetzung nach einem der vorhergehenden Ansprüche, wobei es sich bei dem Modulator des S1P-Rezeptors um 2-Amino-2-[2-(4-octylphenyl)ethyl]propan-1,3-diolhydrochlorid handelt.

6. Zusammensetzung nach Anspruch 1 in Form von Tabletten, Kapseln, Filmtabletten, Lutschtabletten, Pillen, Minitabletten, Kügelchen, Perlen oder Granulat.

7. Zusammensetzung nach Anspruch 1 in Form eines Pulvers, in Form eines Granulats oder in Form von Pellets.

8. Zusammensetzung nach Anspruch 1 in Form einer Tablette.

9. Zusammensetzung nach Anspruch 1 in Form einer Kapsel.

10. Verfahren zur Herstellung einer pharmazeutischen Zusammensetzung nach Anspruch 1, welches die folgenden Schritte umfasst:
(a) das Mischen des Modulators des S1P-Rezeptors mit der mikrokristallinen Cellulose,
(b) das Vermahlen der in (a) erhaltenen Mischung und
(c) das Mischen der in (b) erhaltenen vermahlenen Mischung mit einem Schmiermittel.

## Revendications

1. Composition pharmaceutique solide adaptée pour administration orale, comprenant :
(a) un modulateur de récepteur de S1P ; et
(b) 75 à 99,99 % en poids d'une cellulose microcristalline
en l'absence d'un alcool de sucre,
dans laquelle le modulateur de récepteur de S1P est le 2-amino-2-[2-(4-octylphényl)éthyl]propane-1,3-diol sous forme libre ou sous forme d'un sel pharmaceutiquement acceptable.

2. Composition selon la revendication 1, comprenant 85 à 99,9 % en poids de cellulose microcristalline.

3. Composition selon la revendication 1, comprenant 90 à 99,5 % en poids de la cellulose microcristalline.

4. Composition selon la revendication 1, comprenant 0,5 à 5 % en poids de 2-amino-2-[2-(4-octylphényl)éthyl]propane-1,3-diol, ou un sel pharmaceutiquement acceptable de celui-ci.

5. Composition selon l'une quelconque des revendications précédentes dans laquelle le modulateur de récepteur de S1P est le chlorhydrate de 2-amino-2-[2-(4-octylphényl)éthyl]propane-1,3-diol.

6. Composition selon la revendication 1, sous la forme de comprimés, capsules, cachets, tablettes, pilules, minicomprimés, pastilles, billes ou granules.

7. Composition selon la revendication 1, sous la forme d'une poudre, d'un granule ou de pastilles.

8. Composition selon la revendication 1, sous la forme d'un comprimé.

9. Composition selon la revendication 1, sous la forme d'une capsule.

10. Procédé de production d'une composition pharmaceutique selon la revendication 1, comprenant les étapes de :
(a) mélange du modulateur de récepteur de S1P avec la cellulose microcristalline ;
(b) broyage du mélange obtenu dans (a) ; et
(c) mélange du mélange broyé obtenu dans (b) avec un lubrifiant.
